## Europäisches Patentamt

(19) **European Patent Office**

**Office européen des brevets**

(11) Numéro de publication: **0 192 909**

**B1**

(12) **FASCICULE DE BREVET EUROPÉEN**

(45) Date de publication du fascicule du brevet:
10.05.89

(51) Int. Cl.⁴: **A 61 K 47/00, A 61 K 9/00**

(21) Numéro de dépôt: **85450029.5**

(22) Date de dépôt: **11.12.85**

(54) **Nouveau procédé d'obtention de formes pharmaceutiques à libération prolongée.**

(30) Priorité: **21.01.85 FR 8500678**

(43) Date de publication de la demande:
**03.09.86 Bulletin 86/36**

(45) Mention de la délivrance du brevet:
**10.05.89 Bulletin 89/19**

(84) Etats contractants désignés:
**BE CH DE GB IT LI LU NL**

(56) Documents cité:
**GB-A-857 193**
**GB-A-859 348**
**US-A-2 857 311**
**US-A-3 035 979**

(73) Titulaire: **SOCIETE CORTIAL S.A., 7 rue de l'Armorique, F-75015 Paris (FR)**

(72) Inventeur: **Martani, Rosa, 28 avenue d'Arès, F-33000 Bordeaux (FR)**
Inventeur: **Le Huede, Elisabeth, 36 rue Marceau, F-33200 Bordeaux (FR)**
Inventeur: **Dumas, Jeanne, 31 rue du Docteur Bert, F-33200 Bordeaux (FR)**

(74) Mandataire: **Tajan, Marie-Thérèse, LABORATOIRES SARGET Avenue du Président JF Kennedy, F-33701 Mérignac (FR)**

**Description**

La présente invention concerne un nouveau procédé d'obtention de formes pharmaceutiques à libération prolongée.

Plusieurs brevets ont déjà décrit de nombreux procédés.

- Soit que les principes actifs sont dilués dans un excipient inerte pulvérulent mélange auquel on ajoute un agent tensio-actif (Brevets Français numéros 8 213 613 et 8 204 672).
- Soit qu'ils utilisent le principe d'une matrice enrobée ou non d'un film (Brevets Belge numéro 783 661, Français numéros 8 218 407, 8 221 109, 7 928 056 et 7 630 362, 7 219 993).
- Soit que les principes actifs sont enrobés dans des sphérules ou particules d'un phospholipide (Brevet français numéro 7 602 016)
- Soit que l'on utilise des complexes sans enrobage à partir d'une résine échangeuse d'ions. (Brevets Belge numéro 876 857, Brevet Européen numéro 80 870 032 2, Grande-Bretagne numéros 1 576 016, 982 150, 857 193 et 824 337, Brevet Français numéro 7 230 623)
- Soit que le complexe résine-médicament est traité par un agent de solvatation et pourvu de revêtement perméable à l'eau formant barrière de diffusion. (Brevets francais numéros 7 735 611, U.S.A. numéro 3 138 525, Grande Bretagne numéro 1 218 102)

Toutes ces inventions intéressent principalement les formes galéniques solides. Rares sont celles qui s'adressent à des formes liquides, homogènes.

Nous avons trouvé maintenant un procédé qui permet de réaliser des préparations galéniques à libération prolongée administrées per os, particulièrement stables et dont les cinétiques de dissolution peuvent être modulées à volonté.

Notre procédé met en oeuvre l'emploi simultané de 2 résines à caractère anionique dans des proportions variables; ces résines ont la propriété de fixer des principes actifs pour former des complexes résine-principe actif. Nous utilisons une résine sulfonate et une résine carboxylique.

- La résine sulfonate libère le principe actif lentement: de préférence, il s'agit d'un polymère réticulé contenant des groupements sulfonates et dont l'agent de réticulation est le divinyl-benzène.

Le degré de réticulation est voisin de 8 % et sa granulométrie inférieure à 200 microns.

Le taux de fixation du principe actif sur la résine peut varier de 30 à 70 %.

- La résine carboxylique libère le principe actif beaucoup plus rapidement on peut utiliser de la carboxyméthyl cellulose (échangeur cationique monofonctionnel), mais nous préférons un carbomère (polymère réticulé de l'acide acrylique de haute masse moléculaire et contenant 56 à 68 % de groupe carboxylique).

Pour ce qui est de la résine carboxylique divers brevets (notamment brevet U.S.A. numéro 3 594 470) mentionnent l'utilisation de cette résine carboxylique uniquement dans le but de masquer le goût des principes actifs;

aucun ne fait état de l'utilisation de cette résine développée dans cette invention, où ici elle a deux fonctions:
- Celle de fixer le principe actif
- Celle d'agent de suspension ou de gélifiant lorsqu'il s'agit de formes liquides ou de gel, et celle de matrice lorsqu'il s'agit de formes solides.

Nous avons trouvé:

> > >. d'une part qu'en faisant varier le rapport des taux de principes actifs fixés sur l'une et l'autre résine, il est possible de moduler la cinétique de libération des principes actifs

. d'autre part, qu'en faisant varier la quantité de résine carboxylique il est possible d'obtenir, de par son caractère hydrocolloïde, des préparations pharmaceutiques liquides, des préparations semi-liquides, des gels plus ou moins épais, des comprimés, des poudres (gélules, sachets...).

Les préparations ainsi obtenues sont parfaitement stables et leur cinétique de dissolution n'est pas sensible aux pH.

Ce procédé intéresse tout principe actif ayant un caractère cationique et présentant une demi-vie courte:

Notre étude a porté notamment sur:

> > >- Le chlorhydrate de métoclopramide
- Le bromhydrate de dextrométhorphane
- Les sels de morphine et ses dérivés....

A - <u>Modulation des cinétiques</u>

En faisant varier les taux de principe actif fixés sur chacun des 2 résines nous avons obtenu par la méthode officielle de la palette tournante (note Propharmacopée n° 79), différentes cinétiques de dissolution:

- Exemple du métoclopramide

| TEMPS | A 100% - B 0% (1) | A 75% - B 25% (2) | A 50% - B 50% (3) | A 25% - B 75% (4) | A 0% - B 100% (5) |
|---|---|---|---|---|---|
| 15 minutes | 51 % | 61 % | 70 % | 80 % | 100 % |
| 30 minutes | 55 % | 65 % | 73 % | 83 % | - |
| 1 heure | 61 % | 68 % | 76 % | 85 % | - |
| 2 heures | 65 % | 71 % | 78 % | 88 % | - |

A = Taux de principe actif fixé sur la résine sulfonate
B = Taux de principe actif fixé sur la résine carboxylique

Les résultats de ce tableau sont illustrés par la planche I ci-après qui montre bien la variation des cinétiques selon le rapport des taux de principe actif fixés sur chacune des résines.

Plus le taux fixé sur la résine carboxylique est important plus le taux libéré dès les premières minutes est élevé.

- D'autres principes actifs (codéine, dextrométhorphane, éthylmorphine) imprégnés sur les mêmes résines ont donné les résultats illustrés respectivement par les planches II - III - IV.

On constate que:

Dans les quinze premières minutes la libération du principe actif est fonction de la quantité de principe actif fixée sur la résine carboxylique. Dans les minutes suivantes les courbes de libération ont des pentes voisines.

L'emploi simultané de ces 2 résines permet donc de choisir les doses de principe actif que l'on désire libérer dès les premières minutes, en faisant varier le rapport des quantités fixée sur chacune d'elles.

Par ailleurs, les cinétiques de dissolution ne sont pas influencées par le pH (cf. Planche V illustre le cas du métochlopramide).

B - Obtention des formes galéniques différentes

La résine carboxylique mise en oeuvre, par son caractère hydrocolloïde permet d'obtenir:

| | |
|---|---|
| Des liquides | pour des pourcentages de l'ordre de 0,05 à 0,5 % |
| Des semi-liquides | pour des pourcentages de l'ordre de 0,50 à 1,0 % |
| Des gels | pour des pourcentages de l'ordre de 1 à 2 % |
| Des solides (poudres comp. gélules) | pour des pourcentages de l'ordre de 1 à 10. |

Les modes d'obtention de ces formes galéniques ne présentent pas de difficultés particulières.

Quel que soit le principe actif, le schéma de fabrication reste le même,

- Pour les formes liquides et les gels:

étape 1: imprégnation sur la résine sulfonate de la quantité de principe actif déterminée.

étape 2: solubilisation de la résine carboxylique (carbomère), fixation du principe actif restant à ajouter et incorporation des excipients et de la résine sulfonate imprégnée. La quantité de carbomère mise en oeuvre est fonction de la viscosité recherchée (suspension, gel.....)

Pour les formes solides:

En mélangeant à sec les résines imprégnées à des excipients, on obtient des comprimés ou des poudres présentant des cinétiques variables selon le taux d'imprégnation du principe actif sur chacune des résines.

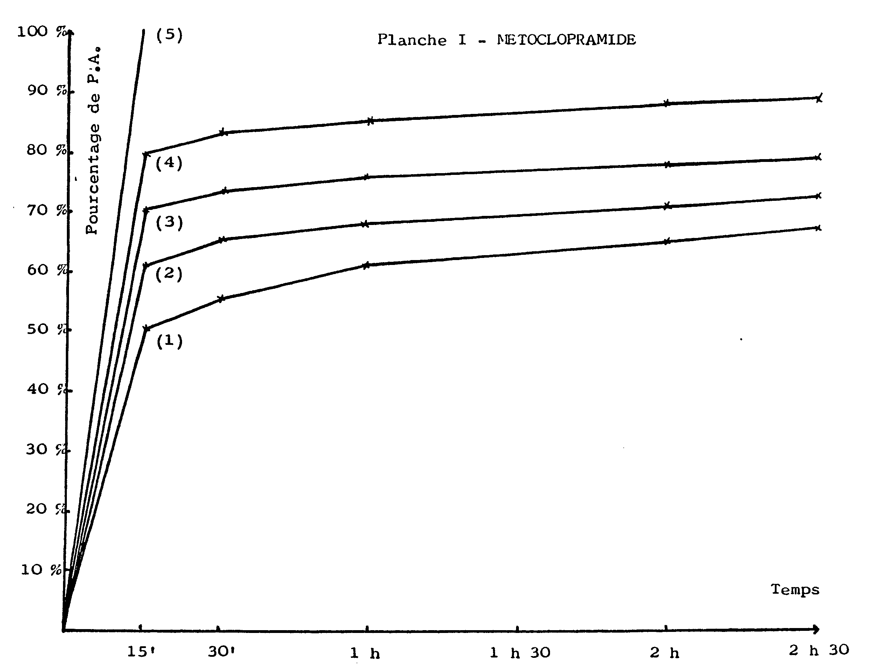

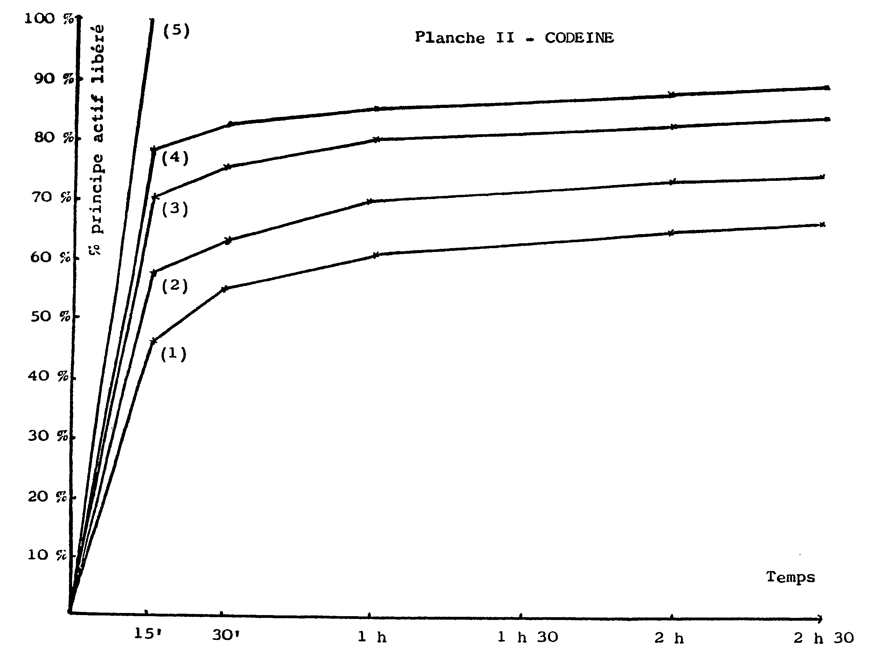

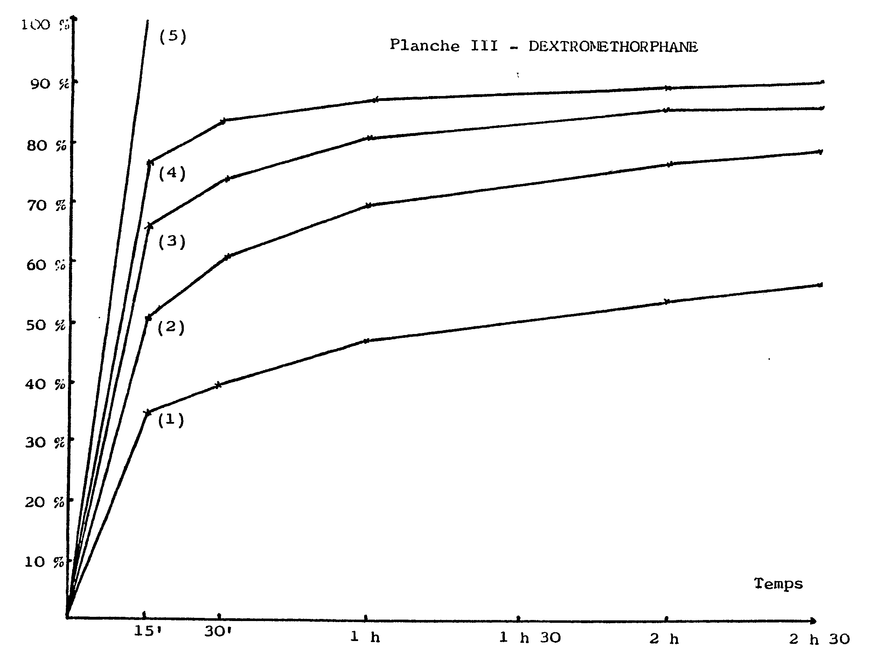

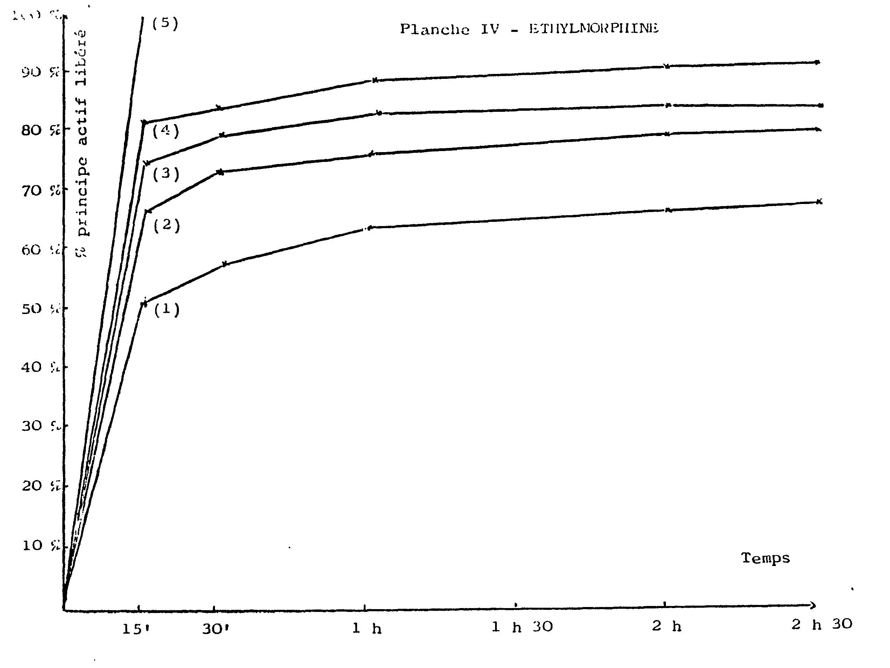

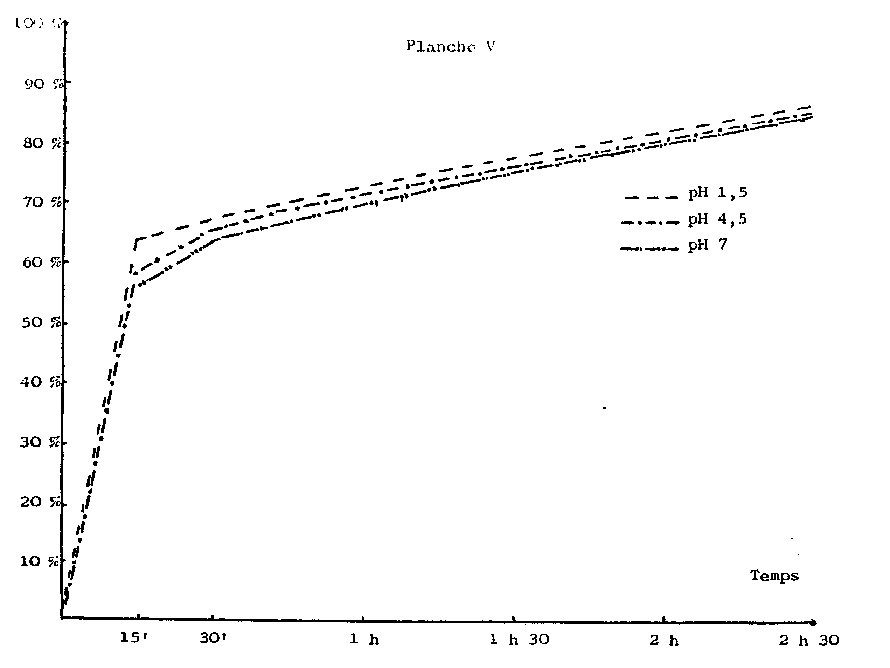

**Revendications**

1. Préparation pharmaceutique stable et orale à libération lente constituée d'un médicament cationique absorbé sur une résine anionique d'échange d'ions se caractérisant par le fait que la résine anionique comprend un composé de résine d'acide sulfonique et de résine d'acide carboxcyliqe hydrocolloïdale où le taux de libération du médicament de la préparation est indépendant du pH et est déterminé par le rapport de poids de la résine d'acide sulfonique et de la résine d'acide carboxylique dans la préparation.

2. Préparation qui, selon la revendication 1, se caractérise par le fait que le médicament cationique est un sel de métoclopramide, codéine, dextrométhorphane, morphine et éthylmorphine.

3. Préparation qui, selon les revendications 1 et 2, se caractérise par le fait que de 30 à 70 % (poids) du médicament cationique est absorbé sur la résine d'acide sulfonique.

4. Préparation qui, selon une des revendications 1 - 3 quelque soit-elle, se caractérise par le fait que la résine d'acide carboxylique comprend un polymère à liaison croisée d'acide acrylique à poids moléculaire élevé contenant 56 - 68 % (poids) de groupes carboxylates.

5. Préparation qui, selon une des revendications 1 - 4 quelque soit-elle, est liquide et se caractérise par le fait que la préparation comprend entre 0,05 et 0,5 % (poids) de résine d'acide carboxylique.

6. Préparation qui, selon une des revendications 1 - 4 quelque soit-elle, est semi-liquide et se caractérise par le fait que la préparation comprend entre 0,5 et 1,0 % (poids) de résine d'acide carboxylique.

7. Préparation qui, selon une des revendications 1 - 3 quelque soit-elle, se présente sous forme de colloïde et se caractérise par le fait que la préparation comprend entre 1 et 2 % (poids) de résine d'acide carboxylique.

8. Préparation qui, selon une des revendications 1 - 4 quelque soit-elle, est solide et se caractérise par le fait que la préparation comprend entre 1 et 10 % (poids) de résine d'acide carboxylique.

9. Préparation qui, selon une revendication quelconque 1 - 4, se caractérise par le fait que la préparation comprend entre 0,05 et 10 % (poids) de résine d'acide carboxylique.

10. Procédé de formulation d'une préparation pharmaceutique selon les revendications 1 - 9 qui se caractérise par l'absorption d'un médicament cationique sur une résine d'acide sulfonique et anionique, l'absorption du médicament cationique sur une résine anionique d'un acide carboxylique hydrocolloïdale et la combinaison des deux résines pour former la préparation où le taux de libération du médicament de la préparation est indépendant du pH et est déterminé par le apport poids de la résine d'acide sulfonique et la résine d'acide carboxylique dans la préparation.

## Claims

1. Stable, oral slow-release pharmaceutical preparation consisting of a cationic medicinal product absorbed on an anionic ion exchange resin, which is characterized in that the anionic resin comprises a mixture of sulphonic acid resin and of hydrocolloidal carboxylic acid resin in which the rate of release of the medicinal product from the preparation is independent of the pH and is determined by the weight ratio of the sulphonic acid resin and of the carboxylic acid resin in the preparation.

2. Preparation which, according to Claim 1, is characterized in that the cationic medicinal product is a salt of metoclopramide, codeine, dextromethorphan, morphine and ethylmorphine.

3. Preparation which, according to Claim 1 or 2, is characterized in that from 30 to 70 % (weight) of the cationic medicinal product is absorbed on the sulphonic acid resin.

4. Preparation which, according to any one of Claims 1 - 3, is characterized in that the carboxylic acid resin comprises a high molecular weight cross linked polymer of acrylic acid containing 56 - 68 % (weight) of carboxylate groups.

5. Preparation which, according to any one of Claims 1 - 4, is liquid and is characterized in that the preparation comprises between 0.05 and 0.5 % (weight) of carboxylic acid resin.

6. Preparation which, according to any one of Claims 1 - 4, is semi-liquid and is characterized in that the preparation comprises between 0.5 and 1.0 % (weight) of carboxylic acid resin.

7. Preparation which, according to any one of Claims 1 - 3, is presented in the form of a colloid and is characterized in that the preparation comprises between 1 and 2 % (weight) of carboxylic acid resin.

8. Preparation which, according to any one of Claims 1 - 4, is solid and is characterized in that the preparation comprises between 1 and 10 % (weight) of carboxylic acid resin.

9. Preparation which, according to any one of Claims 1 - 4, is characterized in that the preparation comprises between 0.05 and 10 % (weight) of carboxylic acid resin.

10. Process for the formulation of a pharmaceutical preparation according to Claims 1 - 9, which is characterized by the absorption of a cationic medicinal product on an anionic and sulphonic acid resin, the absorption of the cationic medicinal product on a hydrocolloidal anionic resin of a carboxylic acid, and the combination of the two resins to form the preparation in which the rate of release of the medicinal product from the preparation is independent of the pH and is determined by ratio of the sulphonic acid resin and the carboxylic acid resin in the preparation.

## Patentansprüche

1. Stabiles orales pharmazeutisches Präparat mit verlängerter Agabe, bestehend aus einem auf einem Anionenaustauscherharz absorbierten kationischen Arzneimittel, dadurch gekennzeichnet, daß das Anionenharz aus einem hydrokolloidalen Sulfonsäureharz/-Carbonsäureharzgemisch besteht, wobei die Abgabegeschwindigkeit des Arzneimittels im Präparat pH-unabhängig und durch das Gewichtsverhältnis des Sulfonsäureharzes zum Carbonsäureharz im Präparat bestimmt ist.

2. Präparat nach Anspruch 1, dadurch gekennzeichnet, daß das kationische Arzneimittel ein Salz von Metoclopramid, Codein, Dextromethorphan, Morphin oder Ethylmorphin ist.

3. Präparat nach Anspruch 1 oder 2, dadurch gekennzeichnet, daß 30 bis 70 Gew.-% kationisches Arzneimittel auf dem Sulfonsäureharz absorbiert sind.

4. Präparat nach einem der Ansprüche 1 - 3, dadurch gekennzeichnet, daß das Carbonsäureharz aus einem hochmolekularen vernetzten Acrylsäurepolymeren mit 56 - 68 Gew.-% Carboxylgruppen besteht.

5. Präparat nach einem der Ansprüche 1 - 4, das flüssig ist, dadurch gekennzeichnet, daß es zwischen 0,05 und 0,5 Gew.-% Carbonsäureharz enthält.

6. Präparat nach einem der Ansprüche 1 - 4, das halbflüssig ist, dadurch gekennzeichnet, daß es zwischen 0,5 und 1,0 Gew.-% Carbonsäureharz enthält.

7. Präparat nach einem der Ansprüche 1 - 3, in Form eines Kolloids, dadurch gekennzeichnet, daß es zwischen 1 und 2 Gew.-% Carbonsäureharz enthält.

8. Präparat nach einem der Ansprüche 1 - 4, das fest ist, dadurch gekennzeichnet, daß es zwischen 1 und 10 Gew.-% Carbonsäureharz enthält.

9. Präparat nach einem der Ansprüche 1 - 4, dadurch gekennzeichnet, daß es zwischen 0,05 und 10 Gew.-% Carbonsäureharz enthält.

10. Verfahren zur Formulierung eines pharmazeutischen Präparats nach einem der Ansprüche 1 - 9, gekennzeichnet durch die Absorption eines kationischen Arzneimittels auf einem anionischen Sulfonsäureharz, Absorption des kationischen Arzneimittels auf einem anionischen hydrokolloidalen Carbonsäureharz und Kombination der beiden Harze zur Bildung des Präparats, wobei die Abgabegeschwindigkeit des Arzneimittels im Präparat pH-unabhängig und durch das Gewichtsverhältnis des Sulfonsäureharzes zum Carbonsäureharz im Präparat bestimmt ist.

Pourcentage de P.A.

100 %

90 %

80 %

70 %

60 %

50 %

40 %

30 %

20 %

10 %

(5)

(4)

(3)

(2)

(1)

15'     30'     1 h     1 h 30     2 h     2 h 30

Temps

Planche I - METOCLOPRAMIDE

1

Planche II - CODEINE

% principe actif libéré

Temps

Planche III – DEXTROMETHORPHANE

Planche IV - ETHYLMORPHINE

Planche V

pH 1,5
pH 4,5
pH 7

Temps

9